# EUROPÄISCHE PATENTANMELDUNG

(11) **EP 2 345 452 A1**
(43) Veröffentlichungstag der Anmeldung: **20.07.2011**
(21) Anmeldenummer: 11150326.4
(22) Anmeldetag: 06.01.2011
(51) Int. Cl.: A61N 1/05, A61N 1/37, A61N 1/08

(54) **Vorrichtung für medizinische Anwendungen**

(30) Priorität: 14.01.2010 US 294864 P
(71) Anmelder: BIOTRONIK SE & Co. KG, 12359 Berlin (DE)
(72) Erfinder: Dörr, Thomas, 12437, Berlin (DE); Weiss, Ingo, 12435, Berlin (DE)
(74) Vertreter: Lindner-Vogt, Karin L.

(57) **Zusammenfassung**

Die vorliegende Erfindung betrifft eine Vorrichtung für medizinische Anwendungen. Die Vorrichtung umfasst ein längliches leitfähiges Element mit einem distalen Ende. In der Nähe des distalen Endes ist ein Widerstandselement angeordnet. Dieses verändert seinen Widerstand in Abhängigkeit einer Temperatur und/oder eines elektromagnetischen Feldes. Dabei ist das Widerstandselement dazu ausgebildet, einen durch Energieaufnahme aus einem elektromagnetischen Feld verursachten Temperaturanstieg des distalen Endes zu verringern.

## Beschreibung

Die vorliegende Erfindung betrifft eine Vorrichtung für medizinische Anwendungen, welche ein längliches leitfähiges Element mit einem distalen Ende umfasst. Ein solches längliches leitfähiges Element kann z. B. eine Leitung sein.

Solche Vorrichtungen werden unter anderem in einer implantierbaren Defibrillationsanordnung 101 verwendet, wie Sie zum Beispiel aus der US 5,531,766 bekannt und in Fig. 1 illustriert ist. Um das Herz H des Patienten P zu stimulieren, steht ein Defibrillator 102 in elektrischer Verbindung mit einer Leitung 103, welche hier eine spezielle Elektrode in Form einer Schockelektrode 104 trägt, die sich an ihrem distalen Ende befindet und im Herzen des Patienten verlegt wird. Die Defibrillation erfolgt in der Regel monopolar über einen Strompfad zwischen dieser Elektrode 104 und einer Gegenelektrode 105, die mehr oder weniger entfernt von dem zu stimulierenden Herzen H liegt. Beispielsweise kann das Gehäuse des Defibrillators 102, das die Einheiten zur Detektion von Herzsignalen und Erzeugung von elektrischen Impulsen enthält, als Gegenelektrode 105 wirken.

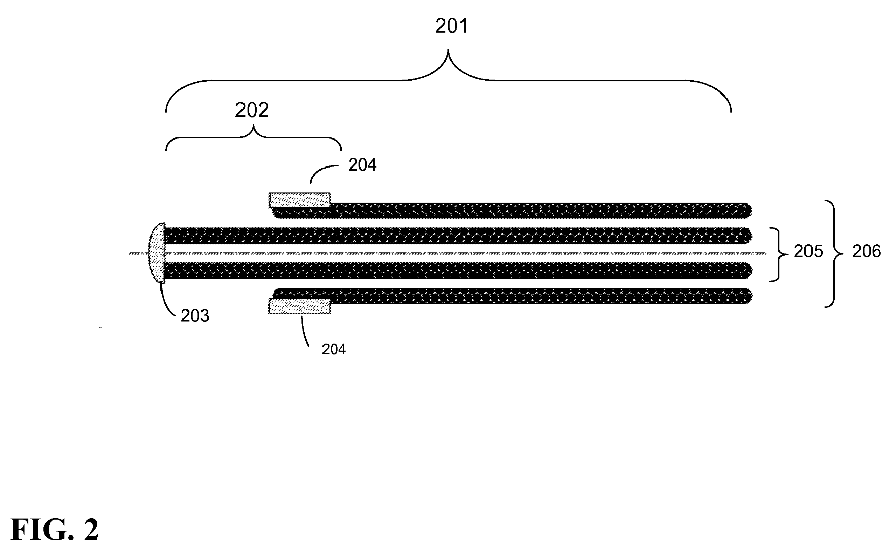

Fig. 2 zeigt in einer anderen möglichen Ausführungsform das distale Ende einer Leitung 201. Die Spitze 202 der Leitung umfasst zur Ausführung einer sogenannten bipolaren Kardioversion zwei Elektroden 203 und 204. Während die Elektrode 203 einen Kontakt an der Spitze der Leitung 201 bildet, ist die Elektrode 204 als Ringkontakt am Umfang der Leitung ausgestaltet. Die Zuleitung umfasst zwei spiralig gewickelte Leiter 205 und 206, die jeweils die Elektroden 203 und 204 elektrisch mit einem (nicht gezeigten) Anschlussstecker am proximalen Ende der Leitung verbinden.

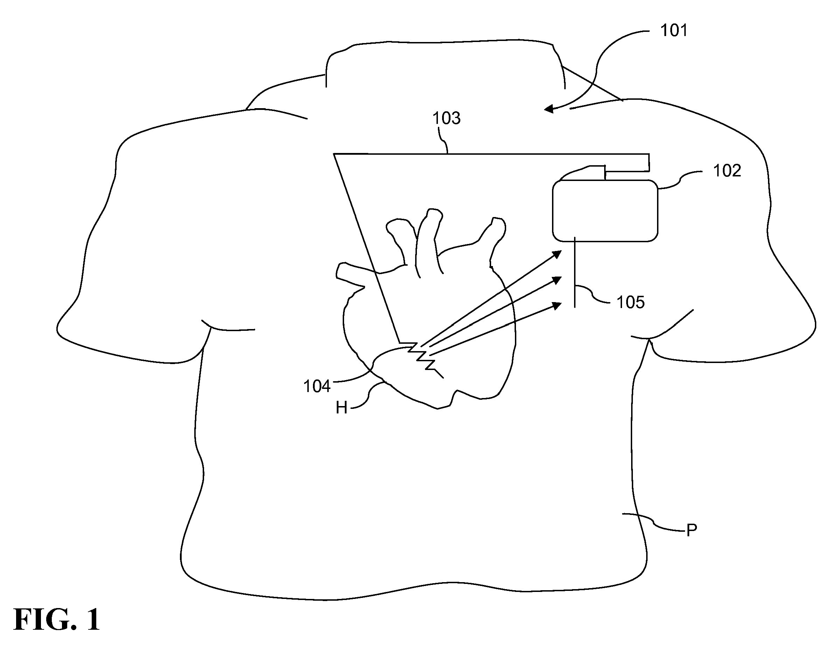

Patienten, die eine konventionelle Defibrillationsanordnung nach Fig. 1 in sich tragen, können derzeit nicht mit Hilfe eines Magnetresonanztomographen (MRT) untersucht werden, da es aufgrund der starken elektromagnetischen Wechselfelder zu einer starken Erwärmung der Leitungsspitze und einer damit zusammenhängenden Schädigung des umliegenden Gewebes kommen kann.

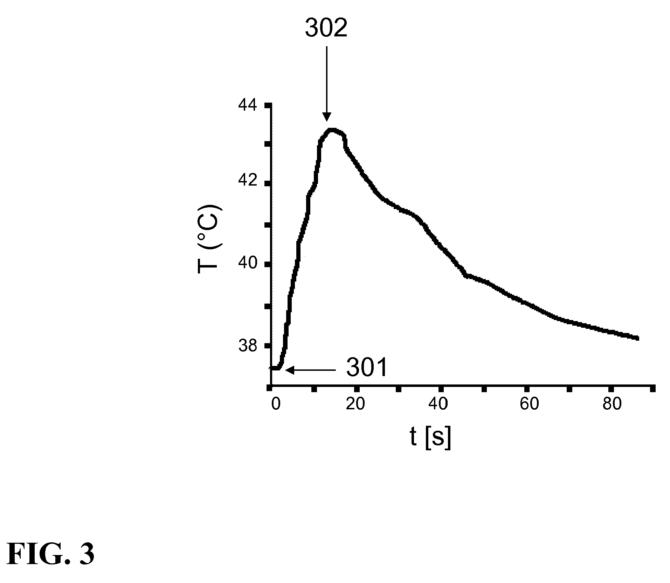

In Fig. 3 ist ein typischer Temperaturverlauf einer Leitungsspitze einer Defibrillationsanordnung in einem Magnetresonanztomographen dargestellt. Mit dem Einschalten des elektromagnetischen Feldes (bezeichnet mit 301) steigt die Temperatur der Leitungsspitze rasch an, wobei die Steilhalt des Anstieges und die maximal auftretende Temperatur stark von der Leitungslage bezogen auf das elektromagnetische Feld des Magnetresonanztomographen abhängig sind. Wird das elektromagnetische Wechselfeld abgeschaltet (bezeichnet mit 302), kühlt sich die Leitungsspitze aufgrund ihrer vergleichsweise geringen Wärmekapazität verhältnismäßig schnell wieder ab.

Zur Lösung des beschriebenen Erhitzungsproblems schlägt die US-Patentanmeldung 2009/0105789 A1 vor, einen temperaturabhängigen Schalter einzusetzen, der bei Erwärmung die Leitungsspitze von der Zuleitung trennt. Ein solcher Schalter führt jedoch zu ungünstigen konstruktiven Eigenschaften der Leitung, insbesondere zu einer Steifheit der Elektrode. Darüber hinaus besitzt der Thermoschalter eine deutliche Hysterese, der Temperaturbereich ist schlecht einstellbar, und die Leitungsspitze kann nur komplett zu- oder abgeschaltet werden, so dass im abgeschalteten Zustand weder eine Stimulation des Herzens noch eine Ableitung von Signalen möglich ist.
Das Überhitzungsproblem der Elektrodenflächen tritt auch bei der temporären Anwendung von Elektroden, elektrisch aktiven Kathetern und metallischen, teilweise isolierten Führungsdrähten im MRT auf. Daher wird die Anwendung des MRT, z.B. im Rahmen elektrophysiologischer Untersuchungen (Mapping) entsprechend eingeschränkt.

### Zusammenfassung der Erfindung

Gemäß einem Aspekt der Erfindung wird eine Vorrichtung für medizinische Anwendungen, die ein längliches leitfähiges Element mit einem distalen Ende umfasst, bereitgestellt, bei der in der Nähe des distalen Endes ein Widerstandselement angeordnet ist, das seinen Widerstand in Abhängigkeit einer Temperatur und/oder eines elektromagnetischen Feldes verändert. Das Widerstandselement ist dazu ausgebildet, einen durch Energieaufnahme aus einem elektromagnetischen Feld verursachten Temperaturanstieg des distalen Endes zu verringern.

Dabei kann das Widerstandselement ein temperaturabhängiger Widerstand sein, dessen Arbeitspunkte z. B. zwischen 38°C und 45°C liegen. Weiterhin kann das Widerstandselement ein magnetfeldabhängiger Widerstand sein, wie z. B. ein GMR (Giant MagnetoResistance)-Element. Auch mittels eines Feldeffekttransistors kann eine Schaltung gebildet sein, die einen veränderlichen Widerstand darstellt.

In einer Ausführungsform ist das Widerstandselement nicht mehr als 20mm von einer Spitze des länglichen leitfähigen Elements entfernt. Das Widerstandselement kann so angeordnet sein, dass eine gute Wärmeleitfähigkeit zwischen der Spitze und dem Widerstandselement besteht, insbesondere kann sich das Widerstandselement unmittelbar an die Spitze anschließen. In einer Ausführungsform ist das Widerstandselement zwischen der Spitze und einer Zuleitung angeordnet.

In einer Ausführungsform ist das Widerstandselement ein Kaltleiter, der so ausgebildet ist, dass er bei Körpertemperatur niederohmig ist und bei einer Temperatur, die oberhalb der Körpertemperatur, aber unterhalb einer gewebeschädigenden Temperatur liegt, hochohmig wird. Während der Einkopplung eines elektromagnetischen Feldes durch den Magnetresonanztomographen wird das distale Ende über den Kaltleiter hochohmig mit dem proximalen Ende des länglichen leitfähigen Elements verbunden, so dass der Temperaturanstieg des distalen Endes nennenswert abnimmt. In einer Ausführungsform ist der Kaltleiter so ausgebildet, dass er bei 37°C niederohmig und bei 44°C hochohmig ist.

Der Kaltleiter kann zumindest teilweise vom distalen Ende umfasst sein. Dabei kann das distale Ende beschichtet sein.

In einer Ausführungsform hat das Widerstandselement die Form eines Zylinders mit einer konzentrischen Bohrung, wobei der Zylinder das längliche leitfähige Element zumindest teilweise bildet oder es umgreift.

Das längliche leitfähige Element kann eine Ableitungseinheit aufweisen, die in der Nähe des distalen Endes angeordnet ist und die aus einem elektromagnetischen Feld aufgenommene Energie von dem distalen Ende wegleitet. Durch das Ableiten der Energie kann eine weitere Reduktion des Temperaturmaximums am distalen Ende erreicht werden.

In einer Ausführungsform umfasst die Ableitungseinheit einen Heißleiter, der so ausgebildet ist, dass er bei Körpertemperatur hochohmig ist und bei einer Temperatur, die oberhalb der Körpertemperatur, aber unterhalb einer gewebeschädigenden Temperatur liegt, niederohmig wird. In einer Ausführungsform ist der Heißleiter so ausgebildet, dass er bei 37°C hochohmig und bei 44°C niederohmig ist, abweichende Werte sind jedoch möglich.

In einer Ausführungsform umfasst das distale Ende einen Elektrodenpol. Dabei kann das längliche leitfähige Element mindestens einen weiteren Elektrodenpol umfassen. Bei dieser Konstellation kann die erfindungsgemäße Vorrichtung dafür eingerichtet sein, Energie aus dem länglichen leitfähigen Element auf die Elektrodenpole möglichst optimal zu verteilen.

In einer Ausführungsform ist die erfindungsgemäße Vorrichtung als Elektrodenleitung ausgebildet. Dabei kann die Vorrichtung eine vom distalen Ende beabstandete Elektrode, insbesondere eine Ringelektrode oder eine Schockwendel einer multipolaren Elektrode, umfassen, auf die insbesondere die Ableitungseinheit die aufgenommene Energie ableitet. Das distale Ende kann eine Elektrode zur Stimulation eines Herzens, eine ICD-Elektrode (ICD = Implantierbarer Kardioverter/Defibrillator), eine transvenöse ICD-Elektrode, eine subkutan implantierbare ICD-Elektrode oder eine Elektrode zur Neurostimulation sein.

Mit Hilfe der erfindungsgemäßen Vorrichtung kann z. B. eine MRT-sichere Elektrodenleitung in einfacher und preiswerter Weise zur Verfügung gestellt werden. Die Leitung bleibt dabei flexibel. Darüber hinaus ist es möglich, bei Verwendung der erfindungsgemäßen Vorrichtung in einem Defibrillator in einem MRT-Umfeld weiterhin effektiv zu stimulieren, so dass auch schrittmacherabhängige Patienten während einer Untersuchung in einem MRT versorgt werden können.

Die erfindungsgemäße Vorrichtung kann als Katheter, insbesondere als Ablations-, Ballon- oder Einführkatheter, als Führungsdraht, als Einführschleuse oder als Stent ausgebildet sein.

Weiterhin umfasst die Erfindung ein Implantat (eine Anordnung) mit einer erfindungsgemäßen Vorrichtung, wobei das distale Ende eine Elektrode zur Stimulation eines Körperteils umfasst und die Elektrode basierend auf einem Stimulationsparameter angesteuert wird. Dabei umfasst das Implantat eine Einrichtung zur Erkennung einer Veränderung des Widerstands des Widerstandselements und eine Einrichtung zur Anpassung des Stimulationsparameters basierend auf der erkannten Veränderung des Widerstands des Widerstandselements. Das Implantat kann insbesondere eine Eingangsstufe mit einem Sensor zum Empfangen von Messsignalen, die den Zustand des zu stimulierenden Körperteils beschreiben, und einer Einrichtung zur Anpassung einer Charakteristik der Eingangsstufe basierend auf der erkannten Veränderung des Widerstands des Widerstandselements umfassen.

Somit kann, wenn sich ein Patient mit Defibrillator in einem Magnetresonanztomographen befindet, z. B. die Stimulationsleistung des Defibrillators an die Situation angepasst werden. Weiterhin kann z. B. die Sensibilität einer Messeinheit, die der Defibrillator verwendet, um sein Stimulationsverhalten zu steuern, angepasst werden.

Weitere Details der Erfindung und entsprechende Ausführungsformen werden im Folgenden anhand der Figuren beschrieben.
- Fig. 1: zeigt einen Grundaufbau einer implantierbaren Defibrillationsanordnung.
- Fig. 2: zeigt eine Ausführungsform eines distalen Endes einer Leitung.
- Fig. 3: illustriert einen Temperaturverlauf einer Spitze einer Leitung während der Einkoppelung eines elektromagnetischen Feldes durch einen Magnetresonanztomographen.
- Fig. 4: zeigt ein Ersatzschaltbild einer Ausführungsform einer erfindungsgemäßen Vorrichtung.
- Fig. 5: zeigt ein Ersatzschaltbild einer weiteren Ausführungsform einer erfindungsgemäßen Vorrichtung.
- Fig. 6: zeigt ein Beispiel eines Widerstandsverlaufes eines Kaltleiters.
- Fig. 7: zeigt eine Ausführungsform einer erfindungsgemäßen Vorrichtung.
- Fig. 8: zeigt eine weitere Ausführungsform einer erfindungsgemäßen Vorrichtung.
- Fig. 9: zeigt eine weitere Ausführungsform einer erfindungsgemäßen Vorrichtung.

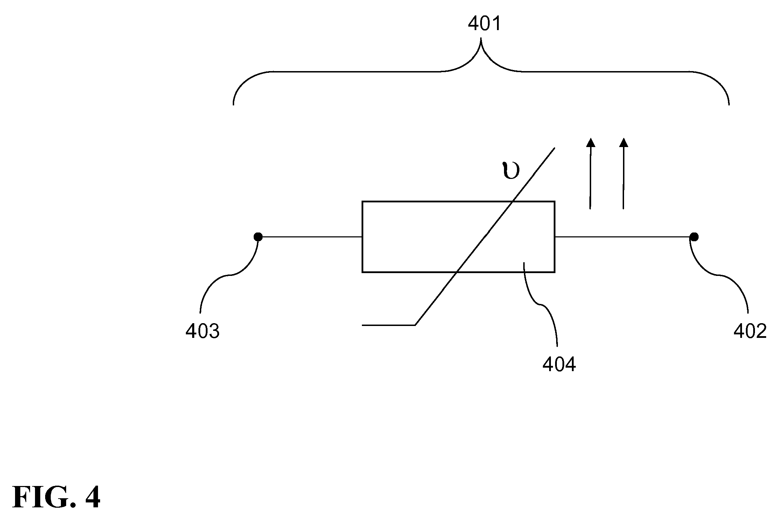

Fig. 4 zeigt ein Ersatzschaltbild einer erfindungsgemäßen Vorrichtung 401, die als Elektrodenleitung ausgebildet ist. Ein längliches leitfähiges Element 402 umfasst ein distales Ende 403 und einen Kaltleiter bzw. PTC-Widerstand 404. Wenn sich das distale Ende 403 während der Einkopplung eines elektromagnetischen Wechselfeldes durch einen MRT erhitzt, wird der in der Nähe des distalen Endes befindliche Kaltleiter 404 erwärmt. Dadurch wird der Kaltleiter 404 hochohmig und reduziert so den Temperaturanstieg.

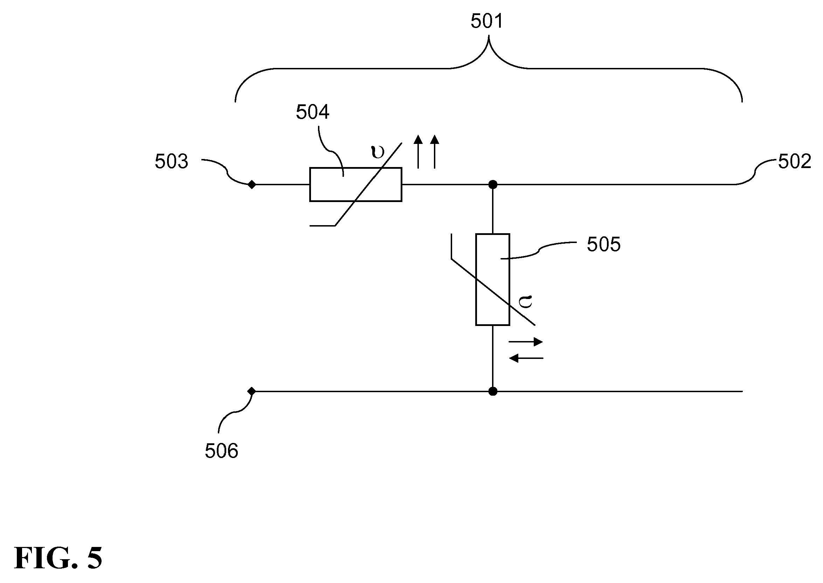

Fig. 5 stellt ein Ersatzschaltbild einer weiteren Ausführungsform einer erfindungsgemäßen Vorrichtung 501 dar. Ein längliches leitfähiges Element 502 umfasst ein distales Ende 503 und einen Kaltleiter 504. Weiterhin weist das längliche leitfähige Element 502 einen Heißleiter bzw. NTC-Widerstand 505 auf. Der Heißleiter 505 verbindet das längliche leitfähige Element 502 mit einer Ringelektrode 506. Mit steigender Temperatur wird der Heißleiter 505 niederohmig und leitet so die aus dem elektromagnetischen Feld aufgenommene Energie von dem distalen Ende 503 auf die thermisch unkritischere Ringelektrode 506 ab.

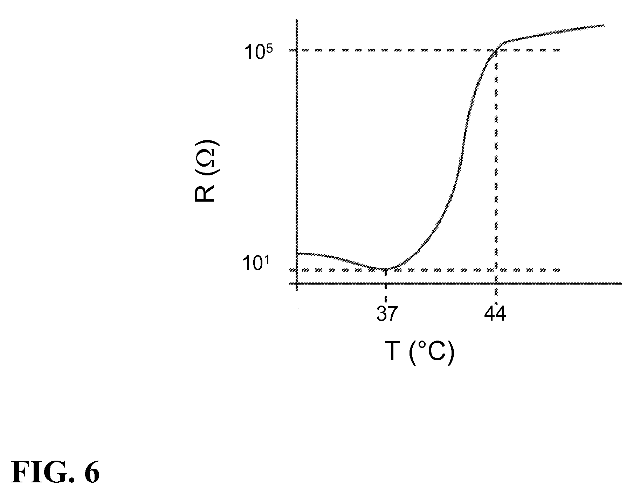

Fig. 6 illustriert einen möglichen Widerstandsverlauf der in Fig. 4 und 5 gezeigten Kaltleiter 404, 504. In dem angegebenen Beispiel liegt der geringste Widerstandswert des Kaltleiters bei 37°C. Mit zunehmender Temperatur steigt der Widerstand des Kaltleiters an und erreicht einen hochohmigen Wert unterhalb der Grenze einer Gewebeschädigung, aber deutlich oberhalb der möglichen Körperkerntemperatur inklusive Fieber. Für den in Fig. 5 gezeigten Heißleiter 505 gilt eine entsprechend umgekehrte Kennlinie.

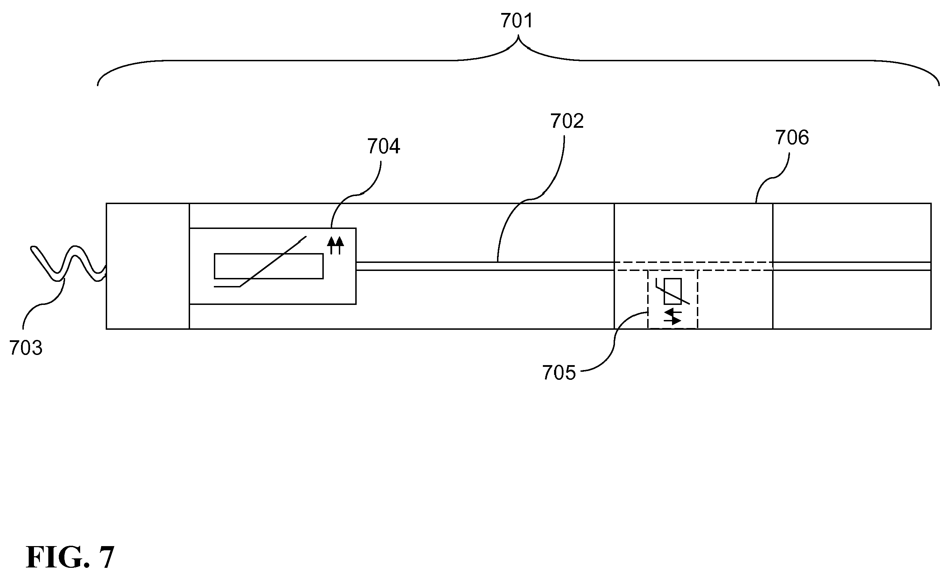

Fig. 7 zeigt eine Ausführungsform einer erfindungsgemäßen Vorrichtung, die als Elektrodenleitung 701 ausgebildet ist. Ein distales Ende 703 des länglichen leitfähigen Elements 702 ist als Schraube ausgeführt und über das längliche leitfähige Element 702 mit einem nicht gezeigten Anschlussstecker verbunden. Erfindungsgemäß ist in der Nähe des distalen Endes 703 ein zylindrischer Kaltleiter 704 montiert, der mit dem länglichen leitfähigen Element 702 verbunden ist. Ein Heißleiter 705 befindet sich zwischen dem länglichen leitfähigen Element 702 und einer Ringelektrode 706.

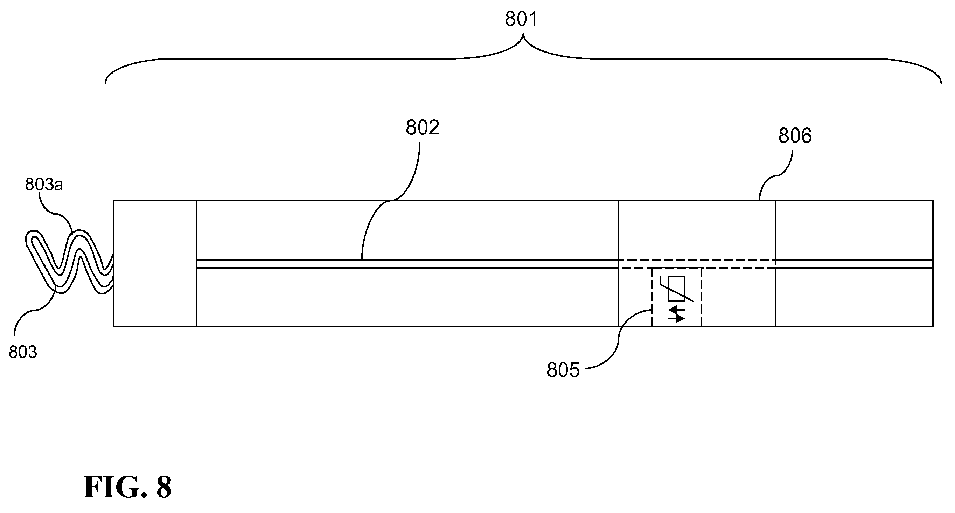

Fig. 8 beschreibt eine weitere Ausführungsform einer erfindungsgemäßen Vorrichtung, die als Elektrodenleitung 801 ausgebildet ist. Ein längliches leitfähiges Element 802, ein Heißleiter 805 und eine Ringelektrode 806 entsprechen dem länglichen leitfähigen Element 702 und der Ringelektrode 706 aus Fig. 7. Abweichend zu der Ausführungsform aus Fig. 7 besteht in Fig. 8 ein distales Ende 803 aus einem Kaltleitermaterial, wie z. B. einem Sinterkörper aus Bariumtitanat, so dass auf einen gesonderten Kaltleiter verzichtet werden kann. Dabei ist das distale Ende 803 mit einer Beschichtung 803a überzogen.

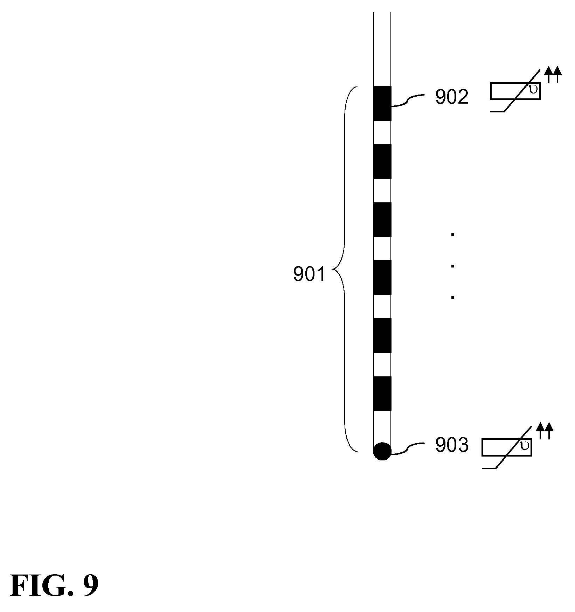

Fig. 9 beschreibt eine weitere Ausführungsform einer erfindungsgemäßen Vorrichtung, die als multipolarer elektrophysiologischer Mappingkatheter 901 ausgebildet ist. Jeder der Ringelektroden 902 und die Tip-Elektrode 903 sind aus einem Kaltleitermaterial gefertigt oder beinhalten einen solchen. Aufgrund der gleichen Elektrodenflächen besteht bei jeder der Ring- bzw. Tip-Elektroden die gleiche Wahrscheinlichkeit, dass diese sich im kritische Bereich erwärmt, so dass eine keine der genannten Elektrode als Gegenelektrode mit einem Heißleiter verschaltet werden kann.

In einer weiteren Ausführungsform der Erfindung umfasst ein Ballonkatheter einen sogenannten Hypotubeschaft, der der Versteifung zur Führung des Katheters dient, aus einem galvanisch leitendem Material besteht und sich vom proximalen Ende bis ca. 40 cm vor den Ballon erstreckt. Erfindungsgemäß ist der Hypotubeschaft als eine erfindungsgemäße Vorrichtung ausgestaltet, um die Erwärmung in einem MRT zu vermindern.

Erfindungsgemäß können auch Ablationskatheter und Einführschleusen, die über Elektroden am distalen Ende verfügen und über eine elektrische Zuleitung mit dem proximalen Ende verbunden sind, wie eine erfindungsgemäße Vorrichtung aufgebaut sein.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Vorrichtung für medizinische Anwendungen, umfassend ein längliches leitfähiges Element mit einem distalen Ende, **gekennzeichnet durch** ein in der Nähe des distalen Endes angeordnetes Widerstandselement, das seinen Widerstand in Abhängigkeit einer Temperatur und/oder eines elektromagnetischen Feldes verändert, wobei das Widerstandselement dazu ausgebildet ist, einen **durch** Energieaufnahme aus einem elektromagnetischen Feld verursachten Temperaturanstieg des distalen Endes zu verringern.

2. Vorrichtung gemäß Anspruch 1, wobei das Widerstandselement ein Kaltleiter ist, der so ausgebildet ist, dass er bei Körpertemperatur niederohmig ist und bei einer Temperatur, die oberhalb der Körpertemperatur, aber unterhalb einer gewebeschädigenden Temperatur liegt, hochohmig wird.

3. Vorrichtung gemäß Anspruch 2, wobei das distale Ende den Kaltleiter zumindest teilweise umfasst.

4. Vorrichtung gemäß Anspruch 1 oder 2, wobei das Widerstandselement die Form eines Zylinders mit einer konzentrischen Bohrung aufweist, der das längliche leitfähige Element zumindest teilweise bildet oder es umgreift.

5. Vorrichtung gemäß einem der vorhergehenden Ansprüche, wobei das längliche leitfähige Element eine Ableitungseinheit aufweist, die in der Nähe des distalen Endes angeordnet ist und die aus einem elektromagnetischen Feld aufgenommene Energie von dem distalen Ende wegleitet.

6. Vorrichtung gemäß Anspruch 5, wobei die Ableitungseinheit einen Heißleiter umfasst, der so ausgebildet ist, dass er bei Körpertemperatur hochohmig ist und bei einer Temperatur, die oberhalb der Körpertemperatur, aber unterhalb einer gewebeschädigenden Temperatur liegt, niederohmig wird.

7. Vorrichtung gemäß einem der vorhergehenden Ansprüche, wobei die Vorrichtung als Elektrodenleitung ausgebildet ist.

8. Vorrichtung gemäß Anspruch 7, wobei die Vorrichtung eine vom distalen Ende beabstandete Elektrode, insbesondere eine Ringelektrode oder eine Schockwendel einer multipolaren Elektrode, umfasst, auf die die Ableitungseinheit die aufgenommene Energie ableitet.

9. Vorrichtung gemäß Anspruch 7 oder 8, wobei das distale Ende eine Elektrode zur Stimulation eines Herzens, eine ICD-Elektrode, eine transvenöse ICD-Elektrode, eine subkutan implantierbare ICD-Elektrode oder eine Elektrode zur Neurostimulation aufweist.

10. Vorrichtung gemäß einem der Ansprüche 1 bis 6, wobei die Vorrichtung als Katheter, insbesondere als Ablations-, Ballon- oder Einführkatheter, ausgebildet ist.

11. Vorrichtung gemäß einem der Ansprüche 1 bis 6, wobei die Vorrichtung als Führungsdraht ausgebildet ist.

12. Vorrichtung gemäß einem der Ansprüche 1 bis 6, wobei die Vorrichtung als Einführschleuse ausgebildet ist.

13. Vorrichtung gemäß einem der Ansprüche 1 bis 6, wobei die Vorrichtung als Stent ausgebildet ist.

14. Implantat mit einer Vorrichtung gemäß einem der Ansprüche 7 bis 9, wobei das distale Ende eine Elektrode zur Stimulation eines Körperteils umfasst und die Elektrode basierend auf einem Stimulationsparameter angesteuert wird und das Implantat eine Einrichtung zur Erkennung einer Veränderung des Widerstands des Widerstandelements und eine Einrichtung zur Anpassung des Stimulationsparameters basierend auf der erkannten Veränderung des Widerstands des Widerstandelements umfasst.

15. Implantat gemäß Anspruch 14, wobei das Implantat weiterhin eine Eingangsstufe mit einem Sensor zum Empfangen von Messsignalen, die den Zustand des zu stimulierenden Körperteils beschreiben, und eine Einrichtung zur Anpassung einer Charakteristik der Eingangsstufe basierend auf der erkannten Veränderung des Widerstands des Widerstandelements umfasst.
